# EUROPEAN PATENT APPLICATION

(11) **EP 4 501 446 A1**
(43) Date of publication of application: **05.02.2025**
(21) Application number: 24160291.1
(22) Date of filing: 28.02.2024
(51) Int. Cl.: B01J 20/22, B01D 53/02, B01J 20/34, B01D 53/04, B01D 53/32

(54) **CARBON DIOXIDE ADSORBENT AND CARBON DIOXIDE ADSORPTION-DESORPTION DEVICE**

(30) Priority: 02.08.2023 JP 2023126393
(71) Applicant: Kabushiki Kaisha Toshiba, Tokyo 105-0023 (JP)
(72) Inventor: SAITO, Hitomi, Tokyo, 105-0023 (JP); NAKANO, Yoshihiko, Tokyo, 105-0023 (JP); YOSHIMURA, Reiko, Tokyo, 105-0023 (JP)
(74) Representative: Marks & Clerk LLP

(57) **Abstract**

According to one approach, a carbon dioxide adsorbent is provided. The carbon dioxide adsorbent is capable of adsorbing and desorbing carbon dioxide and has a fused heteroaromatic ring structure that includes one or more quinone structures.

## Description

### FIELD

Approaches described herein relate generally to a carbon dioxide adsorbent and a carbon dioxide adsorption-desorption device.

### BACKGROUND

A technique for causing carbon dioxide (CO₂) to be absorbed into an absorbent such as an amine is used in a carbon dioxide capture and storage (CCS) plant such as a thermal power plant and is regarded as a most promising candidate as a technique for global warming prevention. The absorbent that has absorbed carbon dioxide is generally heated in a regeneration tower, thereby being regenerated by releasing the carbon dioxide, and repeatedly used. A general temperature for heating there is about 140°C, consuming great energy. Heat and energy required for regeneration are also called heat duty or energy penalty. If this heating temperature can be lowered to efficiently release carbon dioxide, energy reduction can be achieved, allowing promotion of the spread of this technique as a technique for global warming prevention.

As an energy-saving regeneration method, there is a method by which adsorbed carbon dioxide is substituted with water vapor. Carbon dioxide is released from the adsorbent by a substitution reaction of adsorbed carbon dioxide with water vapor. According to this method, the heating temperature can be lowered to 100°C or lower, but water vapor is contained in the released gas in addition to carbon dioxide. Therefore, the process of cooling the released gas, condensing the water vapor, to thereby separate the water vapor from the carbon dioxide is added at a subsequent stage. Therefore, although energy saving can be achieved as compared with thermal desorption, the method can hardly be said to be optimal as a method for regenerating an absorbent.

On the other hand, there has also been studied a technique of separating carbon dioxide by switching between electrical potentials, instead of heating, using molecules or a polymer containing electroactive groups. By using this method, carbon dioxide can be released without heating, so that the effect of energy saving can be expected.

### BRIEF DESCRIPTION OF THE DRAWINGS

FIG. 1 is a CO₂ adsorption/desorption scheme by an example of a redox molecule having a fused heteroaromatic ring structure including a quinone structure.
FIG. 2 is a CO₂ adsorption/desorption scheme by another example of a redox molecule having a fused heteroaromatic ring structure including a quinone structure.
FIG. 3 is a list diagram showing structural formulae of examples of redox molecules having a fused heteroaromatic ring structure including a quinone structure.
FIG. 4 is a list diagram showing structural formulae of other examples of redox molecules having a fused heteroaromatic ring structure including a quinone structure.
FIG. 5 is a list diagram showing structural formulae of other examples of redox molecules having a fused heteroaromatic ring structure including a quinone structure.
FIG. 6 is a distribution diagram showing the CO₂ binding energy at the reaction moiety and the priority of CO₂ adsorption to the reaction moiety as compared with generation of active oxygen species for some examples of oxidation-reduction molecules.
FIG. 7 is a cross-sectional view schematically representing an example of a carbon dioxide adsorption-desorption device according to the approach.
FIG. 8 is a cross-sectional view schematically representing an electrochemical cell that may be included in another example of a carbon dioxide adsorption-desorption device according to the approach.
FIG. 9 is a graph representing a cyclic voltammetry curve measured in Example 1.

### DETAILED DESCRIPTION

According to one approach, a carbon dioxide adsorbent is provided. The carbon dioxide adsorbent is capable of adsorbing and desorbing carbon dioxide and has a fused heteroaromatic ring structure. The fused heteroaromatic ring structure includes one or more quinone structures.

The above approaches provide a carbon dioxide adsorbent that can realize a carbon dioxide adsorption-desorption device that efficiently adsorbs and desorbs carbon dioxide and a carbon dioxide adsorption-desorption that includes the adsorbent.

Hereinafter, approaches will be described with reference to the drawings. The same reference signs are applied to common components throughout the approaches and overlapped explanations are thereby omitted. Each drawing is a schematic view for encouraging explanations of the approach and understanding thereof, and thus there are some details in which a shape, a size and a ratio are different from those in a device actually used, but they can be appropriately design-changed considering the following explanations and known technology.

The approaches described below relate to an adsorbent and a device for separating carbon dioxide (CO₂) from a gas containing the carbon dioxide by an electrochemical process. The adsorbent is one that has been found to be capable of realizing an electrical response device that efficiently adsorbs and desorbs carbon dioxide.

### (Carbon Dioxide Adsorbent)

The carbon dioxide adsorbent according to the approach has a fused heteroaromatic ring structure, when the structure thereof is electrically-neutral. The fused heteroaromatic ring structure contains one or more quinone structures. The carbon dioxide adsorbent can adsorb and desorb carbon dioxide by containing the quinone structure. Hereinafter, the carbon dioxide adsorbent may be simply referred to as "adsorbent". A compound or a material having a fused heteroaromatic ring structure containing a quinone structure can be produced by applying a known method as appropriate.

According to an approach, there is provided an adsorbent for a device that performs gas adsorption and desorption through an electrochemical swing (electro-swing adsorption). The electrochemical swing process is a process of maintaining a specific potential in the forward direction and maintaining a different potential in the reverse direction. In this method, using Red-Ox active groups that can be put into a reduction state at a certain potential and an oxidation state at a different potential makes it possible to adjust affinity for a target adsorption substance. According to the approach, energy is efficiently delivered to act on an electrochemical reaction that results in capture of a target substance (for example, CO₂).

In such an adsorbent, the quinone structure exhibits an oxidation-reduction response. Namely, the quinone structure functions as a Red-Ox active moiety. In the adsorbent, carbon dioxide is adsorbed and desorbed by the oxidation-reduction response of the quinone structure. Specifically, carbon dioxide can be adsorbed to the quinone structure in a reduction state, and the carbon dioxide adsorbed to the quinone structure can be desorbed in an oxidation state.

It has been reported that a typical adsorbent responsive to oxidation-reduction competes with an oxidation-reduction reaction of oxygen depending on the use environment. In an environment where oxygen exists, active oxygen species may be generated by an oxidation-reduction reaction of oxygen, and it is known that the active oxygen species deteriorate the adsorbent. Therefore, it is desirable that the adsorbent can preferentially exchange electrons even in the presence of oxygen, that is, the adsorbent exhibits a higher oxidation-reduction potential than that of oxygen.

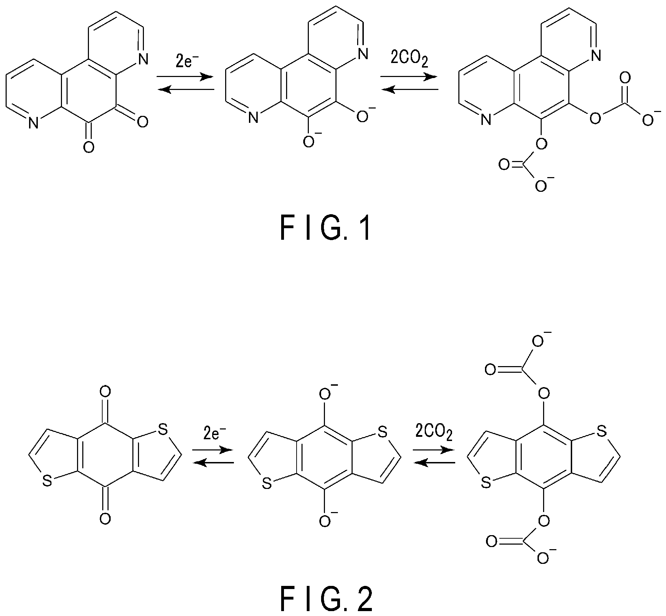

As a result of intensive studies, it was found that the oxidation-reduction potential of a group of molecules having a fused heteroaromatic ring structure containing a quinone structure tends to be higher than the oxidation-reduction potential of oxygen. FIGS. 1 and 2 show examples of carbon dioxide adsorption/desorption schemes by molecules having such a structure. FIG. 1 shows a scheme in the case of using 4,7-phenanthroline-5,6-dione (CAS No. 84-12-8). FIG. 2 shows a scheme in the case of using benzo[1,2-b:4,5-b']bisthiophene-4,8-dione (CAS No. 32281-36-0)_ FIGS. 1 and 2 show examples using redox molecules containing an ortho quinone structure and a para quinone structure, respectively. The quinone structure (diketone structure) gains two electrons when a reduction potential is applied thereto, and binds CO₂ with negatively charged oxygen of ketones, which are negatively charged due to electron localization. Conversely, the bond with CO₂ is resolved by applying an oxidation potential, and a quinone structure is formed again. The above chemical reaction proceeds reversibly.

As an electrochemically active species that can bind to a gas in a system for electrochemically separating a gas such as CO₂, there have been reported an unsubstituted quinone compound such as 1,4-benzoquinone and a substituted quinone compound into which a substituent such as halogen is introduced. Molecules having a fused heteroaromatic ring structure containing a quinone structure are superior in priority against oxygen and CO₂ adsorption during reduction, as compared to the unsubstituted quinone compounds and substituted quinone compounds, which have been conventionally used. Therefore, with the adsorbent containing a fused heteroaromatic ring quinone structure, an electrically responsive carbon dioxide adsorption-desorption device that can efficiently adsorb and desorb carbon dioxide can be provided.

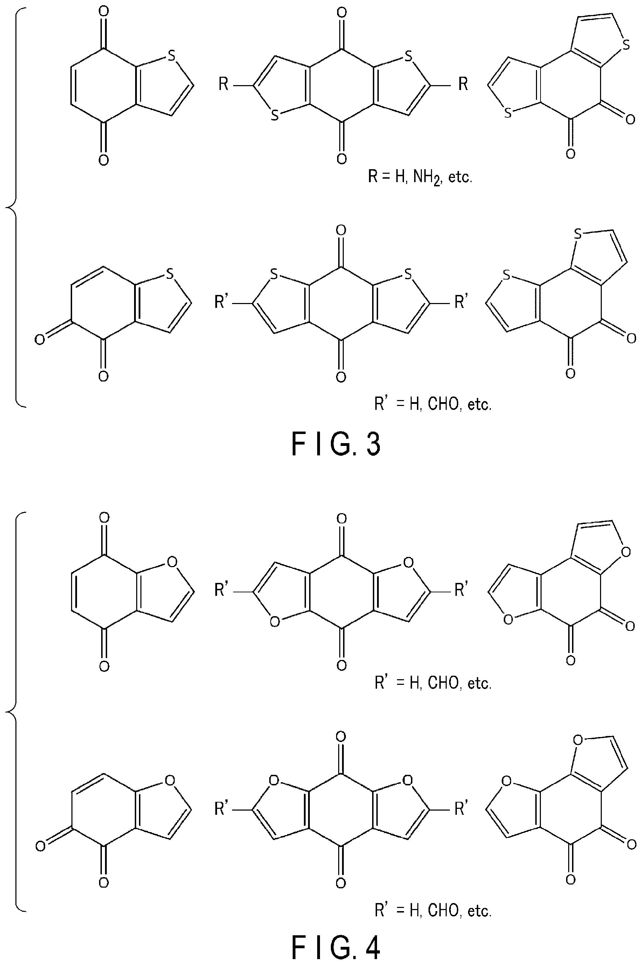

The fused heteroaromatic ring structure desirably has one or more selected from the group consisting of a benzene-based heteroaromatic ring structure, a naphthalene-based heteroaromatic ring structure, an anthracene-based heteroaromatic ring structure, and a phenanthrene-based heteroaromatic ring structure. Namely, the fused heteroaromatic ring structure desirably has a skeleton structure similar to a benzene ring, a naphthalene ring, an anthracene ring, or a phenanthrene ring. The fused heteroaromatic ring structure may be a derivative of a benzene ring, a naphthalene ring, an anthracene ring, or a phenanthrene ring. The heteroatom contained in the fused heteroaromatic ring structure may be one or more selected from the group consisting of nitrogen, oxygen, and sulfur, for example. In addition to nitrogen (N), oxygen (O), and sulfur (S), examples of the heteroatom include phosphorus (P), selenium (Se), and boron (B). The molecule containing a fused heteroaromatic ring structure may have a functional group such as a halogen group, an amino group, a nitro group, an imide group, an imine bond, or an aldehyde group. FIGS. 3 to 5 illustrate examples.

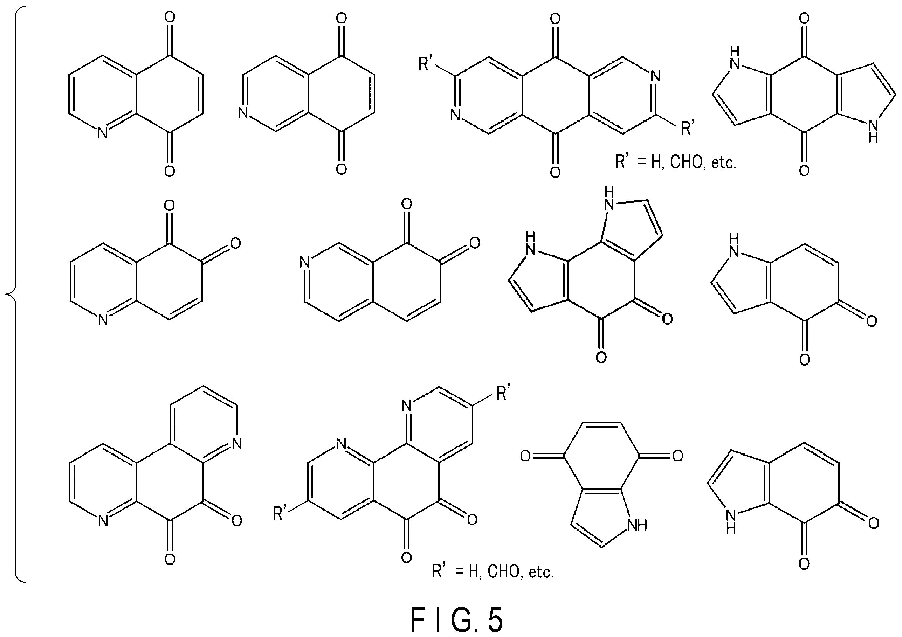

FIGS. 3 to 5 are lists showing structural formulae of some examples of molecules having fused heteroaromatic ring structures containing quinone structures. FIG. 3 shows fused heteroaromatic ring structures containing S as a heteroatom, such as a thiophene-based heteroaromatic ring structure. FIG. 4 shows fused heteroaromatic ring structures containing O as a heteroatom, such as a furan-based heteroaromatic ring structure. FIG. 5 shows examples of molecules having a fused heteroaromatic ring structure containing N as a heteroatom, such as a pyridine-based one and a pyrrole-based one. The fused heteroaromatic ring quinone molecule that can function as the adsorbent is not limited to the illustrated examples. In addition, the fused heteroaromatic ring structure containing a quinone structure that can be contained in the adsorbent is not limited to the structures that the illustrated molecules have.

FIG. 3 shows examples of a thiophene-based molecule having a naphthalene-based heteroaromatic ring structure in the left column. Similarly, FIG. 4 shows examples of a furan-based molecule having a naphthalene-based heteroaromatic ring structure in the left column. FIG. 5 shows four examples of pyridine-based molecules having a naphthalene-based heteroaromatic ring structure in the upper two rows of the left two columns, and three examples of pyrrole-based molecules having a naphthalene-based heteroaromatic ring structure in the lower two rows in the right column and in the bottom row of the middle-right column. These molecules having a naphthalene-based heteroaromatic ring structure can also be described as being molecules having a fused heteroaromatic ring structure that are based on naphthoquinone.

FIG. 3 shows examples of a thiophene-based molecule having an anthracene-based heteroaromatic ring structure in the middle column, FIG. 4 shows examples of a furan-based molecule having an anthracene-based heteroaromatic ring structure in the middle column, FIG. 5 shows an example of a pyrrole-based molecule having an anthracene-based heteroaromatic ring structure in the top row at the right end, and an example of a pyridine-based molecule having an anthracene-based heteroaromatic ring structure neighboring on the left, in the top row of the middle-right column. These molecules having an anthracene-based heteroaromatic ring structure can also be described as being molecules having a fused heteroaromatic ring structure based on anthraquinone.

FIG. 3 shows examples of a thiophene-based molecule having a phenanthrene-based heteroaromatic ring structure in the right column, FIG. 4 shows examples of a furan-based molecule having a phenanthrene-based heteroaromatic ring structure in the right column, FIG. 5 shows two examples of a pyridine-based molecule having a phenanthrene-based heteroaromatic ring structure in the bottom row of the left two columns, and one example of a pyrrole-based molecule having a phenanthrene-based heteroaromatic ring structure in the middle row of the middle-right column. These molecules having a phenanthrene-based heteroaromatic ring structure can also be described as being molecules having a fused heteroaromatic ring structure based on phenanthrenequinone.

Preferably, the Red-Ox active moiety contained in the adsorbent can be reduced in preference over oxygen so as to adsorb CO₂ even in the presence of oxygen. Not only can competition with the oxidation-reduction reaction of oxygen but also deterioration of the adsorbent due to active oxygen species can be avoided, making it possible to select the application environment of the adsorbent and the gas to be treated without being concerned about the presence or absence of oxygen. A material that has high CO₂ binding energy and for which the highest occupied molecular orbital (HOMO) of a molecule in a reduction state is lower than oxygen can be preferentially reduced and adsorb CO₂ in the presence of oxygen. Therefore, evaluation of a molecule that is advantageous in the presence of oxygen as a Red-Ox active moiety is possible, through assessment by first principle calculation of the two evaluation parameters of priority against active oxygen species generation and CO₂ binding energy.

In the quinone structure (diketone structure), oxygen of two ketones (R₂C=O) becomes an anion (R₂C-O⁻) by two-electron reduction, and CO₂ binds to each -O⁻ group to form two R₂C-O-CO₂⁻ groups. Conversely, if the oxygen molecule O₂ undergoes one-electron reduction, it becomes an active oxygen species O₂⁻ (superoxide). Therefore, the difference between the stabilization energy generated when two molecules of O₂⁻ are produced from two molecules of O₂ and the stabilization energy generated when electrons are localized in the quinone structure and oxygen is ionized, is used as the parameter for priority against active oxygen species generation. In addition, the stabilization energy generated by the formation of a bond with CO₂ in a state where the oxygen of a quinone structure is ionized is taken as the CO₂ binding energy, the other parameter. An example is shown below.

The structures and energies of various oxidation-reduction responsive molecules and the interaction/adsorption reaction system between the molecules and CO₂ were calculated by density functional theory (DFT) using B3LYP functional. As the basis function, 6-31++G(d,p) was used. Regarding the molecular structure, structure optimization calculation was performed using an energy gradient method in a dielectric field of acetonitrile, and the structure obtained by reference vibration analysis was confirmed to be an equilibrium structure. For the calculation in the dielectric field, a dielectric model (polarized continuous body (PCM) model), which takes a solvent effect into consideration, was used. All the calculations were performed using a molecular orbital calculation program Gaussian 16 (developed by Hulinks Inc.).

If the CO₂ binding energy is small, achieved is a state equivalent to the CO₂ wandering around the molecule. Therefore, the larger the energy, the more stably the bond between the molecule and CO₂ can be maintained. In addition, the smaller the priority parameter, the difference between the achievement of the reduction state of the molecule and the generation of active oxygen species becomes more ambiguous. Therefore, it is more preferable that the energy difference is larger.

The calculation results of examples of molecules having a fused heteroaromatic ring structure including a quinone structure are shown in Tables 1 to 4. Table 1 shows the calculation results of thiophene-based molecules, Table 2 shows the calculation results of furan-based molecules, Table 3 shows the calculation results of pyridine-based molecules, and Table 4 shows the calculation results of pyrrole-based molecules.

**[Table 1]**

| Thiophene-based molecules having a fused heteroaromatic ring structure including a quinone structure | | |
|---|---|---|
| Chemical structure | Priority over active oxygen | Binding energy to CO₂ (kJ/mol) |
| 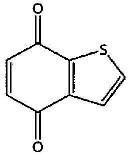 | 21.49 | 32.08 |
| 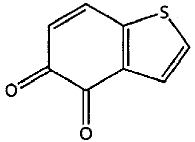 | 25.76 | 34.75 |
| 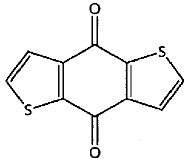 | 12.51 | 26.29 |
| 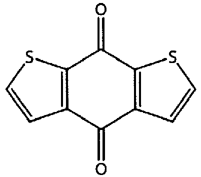 | 12.41 | 26.00 |
| 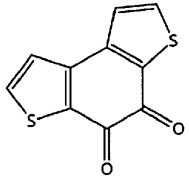 | 18.23 | 27.28 |
| 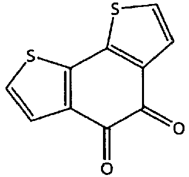 | 20.07 | 33.17 |

**[Table 2]**

| Furan-based molecules having a fused heteroaromatic ring structure including a quinone structure | | |
|---|---|---|
| Chemical structure | Priority over active oxygen | Binding energy to CO₂ (kJ/mol) |
| 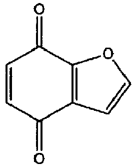 | 23.00 | 33.90 |
| 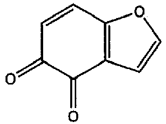 | 26.56 | 36.32 |
| 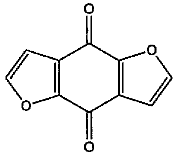 | 14.74 | 30.21 |
| 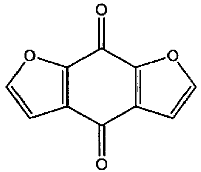 | 14.63 | 29.89 |
| 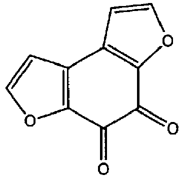 | 19.88 | 32.72 |
| 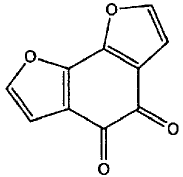 | 20.69 | 36.95 |

**[Table 3]**

| Pyridine-based molecules having a fused heteroaromatic ring structure including a quinone structure | | |
|---|---|---|
| Chemical structure | Priority over active oxygen | Binding energy to CO₂ (kJ/mol) |
| 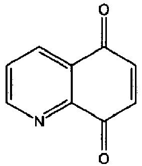 | 21.40 | 32.77 |
| 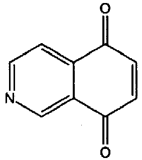 | 21.93 | 29.67 |
| 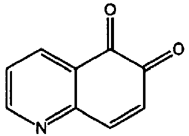 | 25.02 | 31.31 |
| 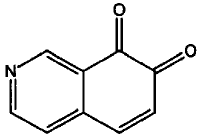 | 25.20 | 29.19 |
| 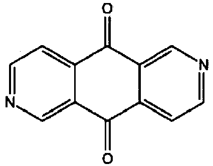 | 10.66 | 20.31 |
| 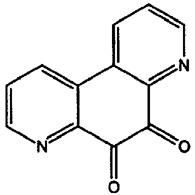 | 22.85 | 30.56 |
| 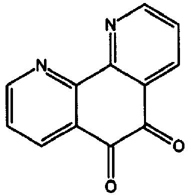 | 20.66 | 29.03 |

**[Table 4]**

| Pyrrole-based molecules having a fused heteroaromatic ring structure including a quinone structure | | |
|---|---|---|
| Chemical structure | Priority over active oxygen | Binding energy to CO₂ (kJ/mol) |
| 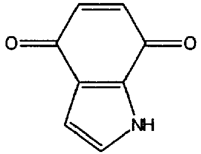 | 14.79 | 37.56 |
| 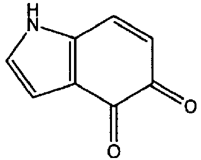 | 19.29 | 41.98 |
| 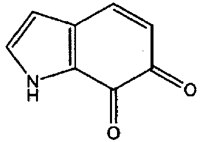 | 18.24 | 39.57 |
| 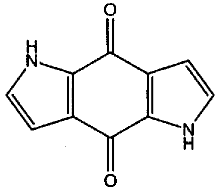 | -3.30 | 38.36 |
| 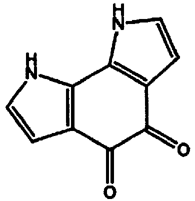 | 8.38 | 47.49 |

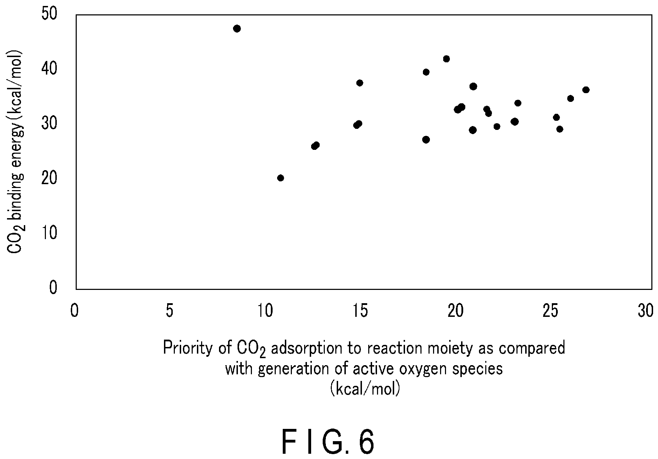

A part of the results shown in Tables 1 to 4 are summarized in FIG. 6. FIG. 6 is a distribution diagram in which the priority against the generation of reactive oxygen species is plotted on the horizontal axis and the binding energy between the molecule and CO₂ is plotted on the vertical axis. Molecules with a higher degree of CO₂ adsorption than active oxygen are more likely to bind with CO₂ even in the presence of oxygen, and molecules with higher CO₂ binding energy are more likely to bind with CO₂.

The heteroatom introduced into a heteroaromatic ring is electron-donating. Therefore, in the quinone compound containing a heteroaromatic ring, the electron density of the O atoms of the quinone somewhat increases, although some differences in magnitude may arise due to conjugation effects (resonance effects) depending on the positional relationship between the heteroatom and the quinone, and thus, the priority against active oxygenation is expected to decrease. However, the priority against active oxygen is high, according to the above calculation results, suggesting that electrons are attracted by heteroatoms, reducing the electron density of the O atoms of quinone. Namely, contrary to the expectation that electron-donating heteroatoms adversely affect the redox of quinone, the introduction of heteroatoms is advantageous.

In the fused heteroaromatic cyclic quinone, electrons are somewhat drawn to the introduced heteroatoms as compared with a quinone that is not heterocyclic but is one where an aromatic ring of a carbon skeleton is provided with diketone, and thus, the degree of electron localization to O atoms of the quinone is lowered. On one hand, since electrons are attracted by heteroatoms, the CO₂ binding energy decreases. On the other hand, since the electron localization to the O atoms of the quinone is reduced, the bonding with CO₂ by the quinone has priority against the activation of the environmental oxygen into active oxygen. Specifically, since the oxidation-reduction potential shifts to a high potential as the electron density decreases, the reaction proceeds with higher priority than active oxygen generation. Therefore, as a result, the heteroaromatic ring is superior.

Even if a halogen substituent is introduced, electrons are drawn by the inductive effect (I effect) of halogen, so that the electron density of O atoms of the quinone decreases. Accordingly, the CO₂ binding energy decreases, but bonding with CO₂ is prioritized over generation of active oxygen. Since the I effect is not included in the conjugated system of the aromatic ring, the effect resulting from halogen introduction is solely the I effect. Fused heteroaromatic ring quinones exhibit more favorable priority against oxygen and more favorable CO₂ adsorption than non-heterocyclic quinones that are substituted with halogen or the like. Therefore, a fused heteroaromatic ring structure including a quinone structure is suitable as a carbon dioxide adsorbent.

The adsorbent may be a porous material including a fused heteroaromatic ring structure containing a quinone structure as a Red-Ox active moiety. In addition, the adsorbent may be an oligomer or a polymer including molecules containing a fused heteroaromatic ring structure as a unit structure.

The porous material, which is one of the possible forms of the carbon dioxide adsorbent, includes a porous material having a large number of pores. The pore diameter of the pores is preferably 5 nm or less. Specifically, the porous material is preferably formed of a subnano-porous material or a nano-porous material having pores of an angstrom size (1 nm or less) to a nanometer size, and more specifically, 0.5 nm or more and 5 nm or less.

The porous material includes a Red-Ox active moiety in which the diketone structure is reduced by an electrical response to bond with carbon dioxide and that is oxidized by an electrical response to resolve the bond with carbon dioxide and re-form the diketone bond, that is, the above-described quinone structure. The Red-Ox active moiety may be contained as a functional group in the molecular structure constituting the porous material. The Red-Ox active moiety is not limited to the molecules and compounds described above as examples, and derivatives thereof and other molecules and compounds having a fused heteroaromatic ring structure including a quinone structure can also be used.

The content of the molecule having the fused heteroaromatic ring structure including the quinone structure exhibiting redox activity according to electrical response contained in the porous material is preferably 10 mass% or more to 80 mass% or less, and more preferably 20 mass% or more to 75 mass% or less, with respect to the total mass of the porous material.

In general, the larger the amount of the active groups contained in the adsorbent, the greater the adsorption amount and desorption amount of carbon dioxide per unit volume of the adsorbent. Therefore, from the viewpoint of energy consumption, the size of plant equipment, and treatment efficiency, it is desirable to have a large amount of active groups.

The content of the molecule having the fused heteroaromatic ring structure including the quinone structure exhibiting redox activity due to electrical response is preferably 90 mass% or less with respect to the total mass of the porous material. With this content, the amount of the Red-Ox active moiety per unit volume of the porous material can be made appropriate, so that the Red-Ox active groups are not excessively close to each other, and pores can be maintained. In addition, since carbon dioxide can easily access the active group, the active group can be sufficiently utilized. The content of the molecule having the fused heteroaromatic ring structure including the quinone structure is more preferably 75 mass% or less.

By setting the content of the Red-Ox active moiety to 10 mass% or more, sufficient adsorption amount and adsorption rate of carbon dioxide can be obtained, and excellent treatment efficiency can be obtained. Therefore, the porous material in which the content of the Red-Ox active moiety is within the above range is advantageous for use in carbon dioxide recovery in that, since the porous material has not only a large carbon dioxide adsorption amount and a high carbon dioxide adsorption rate, but also has a large carbon dioxide desorption amount and a high carbon dioxide desorption rate (reaction rate), whereby recovery of carbon dioxide can be performed efficiently.

Specific examples of the molecule having the fused heteroaromatic ring structure including the quinone structure capable of functioning as the Red-Ox active moiety in the porous material include molecules having an amino group or an aldehyde group as the functional group R or R', as illustrated in FIGS. 3 to 5. Molecules having an amino group or an aldehyde group as the functional group R or R' present at symmetrical positions of the ring as illustrated can be suitably incorporated into the molecular structure of the porous material.

As in the above example, the quinone structures as active reaction points in the porous material are regenerated by desorbing CO₂ after adsorbing CO₂ and repeatedly used. Therefore, the chemical stability of the fused heteroaromatic ring structure including the quinone structure is preferably high.

The porous material preferably contains one or more porous substances having electroactivity selected from the group consisting of a covalent-organic framework (COF), a metal-organic framework (MOF), a porous carbon material, zeolite, porous silica, and porous alumina. For example, the porous material may include a COF having electrical responsiveness. Alternatively, the porous material may contain an MOF having electrical responsiveness.

To determine whether a quinone structure is present or absent, structures that form a fused heteroaromatic ring structure including a quinone structure can be estimated based on ¹H nuclear magnetic resonance (NMR) and ¹³C NMR measurement. If absorption derived from carbonyl stretching vibration is observed at 1600 cm⁻¹ to 1800 cm⁻¹ according to infrared spectroscopy, it can be determined that a ketone structure is included. With regard to a heteroaromatic ring structure, for example, if sulfur atoms are included, strong emission derived from stretching vibration of an aromatic S-C bond is observed at 1060 cm⁻¹ to 1100 cm⁻¹ according to Raman spectroscopy, so that it can be determined that a heteroaromatic ring structure is included. The same applies to a heteroaromatic ring containing nitrogen or oxygen. Thus, infrared spectroscopy, Raman spectroscopy, and NMR spectrometry are combined to examine whether the carbon dioxide adsorbent has a fused heteroaromatic ring structure including a quinone structure.

The carbon dioxide adsorbent described above includes a fused heteroaromatic ring structure including one or more quinone structures. The adsorbent can efficiently adsorb and desorb carbon dioxide even in the presence of oxygen.

### (Carbon Dioxide Adsorption-desorption Device)

The carbon dioxide adsorption-desorption device according to the approach includes the carbon dioxide adsorbent according to the above-described approach.

As described above, the adsorbent can exhibit redox activity by electrical response. Therefore, the carbon dioxide adsorption-desorption device can adsorb and desorb carbon dioxide to and from the Red-Ox active moiety (quinone structure; ketone group) of the adsorbent by a change in potential applied to the device. Such a device using the adsorbent that adsorbs and desorbs carbon dioxide with the electrically responsive Red-Ox active moiety can adsorb and desorb carbon dioxide at room temperature. Therefore, the device can adsorb and desorb carbon dioxide with low energy.

Such a device can suitably adsorb carbon dioxide as described above and can be suitably applied to an apparatus for recovering carbon dioxide from factory exhaust gases or the atmosphere. For example, a carbon dioxide recovery system can be constructed using the carbon dioxide adsorption-desorption device.

Such a device may include an electrode for applying a charge to the Red-Ox active moiety of the adsorbent having the fused heteroaromatic ring structure including the quinone structure. In the device, the adsorbent may be included in the electrode (working electrode). The electrode preferably has a porous form such as a mesh shape so that more adsorbent can exchange electrons. The adsorbent can also be dispersed inside the porous electrode. The adsorbent may be dispersed in a solution within the device. The solution containing the adsorbent and the electrode may be arranged such that the solution and the electrode are in electrical contact with each other in the device.

The electrode may further include a current collector. For example, the adsorbent may be supported on the current collector and included in the electrode in the form of the porous material described above, in a state of being dispersed in another porous structure, or in a state of modifying an electrically conductive member. For example, the porous material may be formed onto the surface of the current collector.

The current collector can function as a conductor for transferring charge to the adsorbent to cause the Red-Ox active moiety to electrically respond. By altering the potential of the current collector, the Red-Ox active moiety included in the adsorbent can be oxidized and reduced, and carbon dioxide can be adsorbed to and desorbed from the moiety.

The porous material and the electro-conductive member can be made into a composite so as to yield a porous composite. The composite can be obtained, for example, by mixing the porous material and the electro-conductive member or modifying (covering) the surface of the electro-conductive member with the porous material.

In the porous composite, the Red-Ox active moiety contained in the adsorbent is preferably close to or in contact with a nearby electro-conductive member. Therefore, it is preferable that the adsorbent covers at least a part of the surface of the electro-conductive member, or that the adsorbent is attached to the electro-conductive member. For example, the above-described COF or MOF may be contained in the porous composite in a manner of covering the electro-conductive member.

The electrode may further include a binder. The binder may be included, or not. The binder can bind the current collector, the porous material, and the electro-conductive member, or bind the current collector and the porous composite, for example. As the binder, for example, polyvinylidene fluoride (PVdF), polymethyl methacrylate (PMMA), or polyimide (PI) may be used.

The carbon dioxide adsorption-desorption device may further include an electrolyte (for example, an electrolytic solution). The adsorbent may be dispersed in the electrolyte solution. Alternatively, the electrolyte may be held in the porous material by the electrode including the porous material or the porous composite. The electrolyte may be contained in pores of the porous material, for example. By including the electrolyte, electrical conductivity is improved, and the ability to adsorb and desorb CO₂ is enhanced.

In the device, an electrode containing the adsorbent or an electrode in electrical contact with a solution in which the adsorbent is dispersed is used as a working electrode. By applying a potential to the working electrode, a charge is applied to the Red-Ox active moiety included in the adsorbent. As such, by controlling the potential of the electrode, the redox state of the adsorbent is switched between the oxidation state and the reduction state. The adsorbent can adsorb carbon dioxide in the reduction state and can desorb carbon dioxide in the oxidation state.

For example, the potential can be applied to the working electrode by using a counter electrode to the working electrode and applying a voltage between these electrodes. For example, the carbon dioxide adsorption-desorption device may include a counter electrode to the electrode included in the device. Namely, as the carbon dioxide adsorption-desorption device, an electrochemical cell using the above working electrode and counter electrode may be configured. Aspects of the device are not limited to electrochemical cells. A specific example of the electrochemical cell is a three-electrode cell further including a reference electrode in addition to the working electrode and the counter electrode.

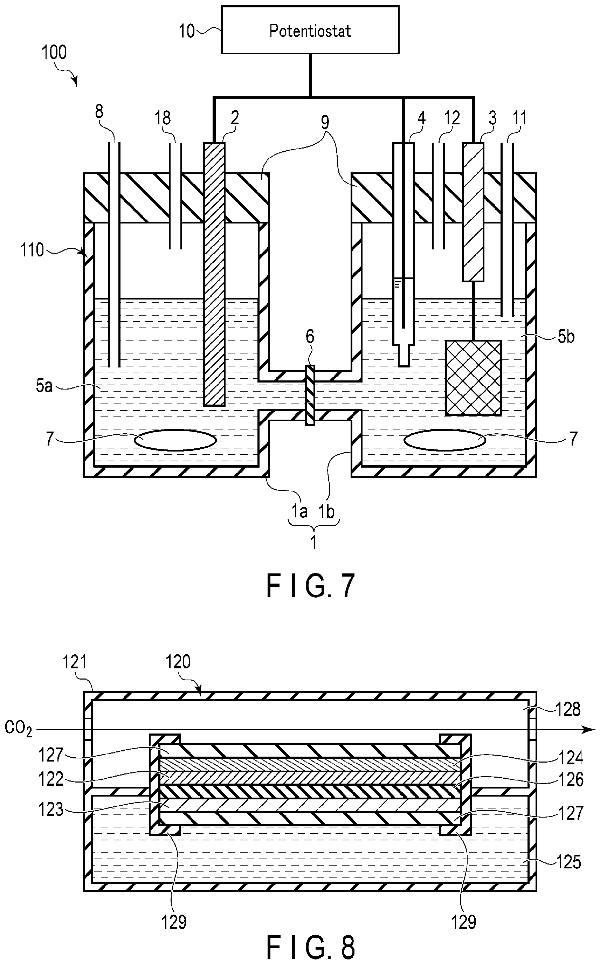

A specific example of a carbon dioxide adsorption-desorption device according to the approach will be described with reference to FIGS. 7 and 8. FIG. 7 is a schematic cross-sectional view of an example of a carbon dioxide adsorption-desorption device according to the approach. FIG. 8 is a schematic cross-sectional view of an electrochemical cell that another example of the device may include.

A carbon dioxide adsorption-desorption device 100 illustrated in FIG. 7 includes an electrochemical cell 110 and a potentiostat 10. The electrochemical cell 110 includes an electrolytic cell 1 including cells 1a and 1b, a working electrode 2, a counter electrode 3, a reference electrode 4, a separator 6, a gas introduction nozzle 8, a gas-flushing inlet 11, a gas-flushing outlet 12, a nozzle 18 for recovering desorbed carbon dioxide, and lids 9. The cell 1a contains an electrolytic solution 5a and a stirrer 7 that stirs the electrolytic solution 5a. The carbon dioxide adsorbent may be contained in the electrochemical cell 110 in a state of being dispersed in the electrolytic solution 5a. The cell 1b contains an electrolytic solution 5b and a stirrer 7 that stirs the electrolytic solution 5b. In one example, the electrolytic solution 5b does not contain the carbon dioxide adsorbent. The lid 9 for preventing intrusion of outside air into the cells 1a and 1b is provided on the opening of each cell, and a gas containing carbon dioxide can be introduced into the electrolytic solution 5a through the nozzle 8 penetrating the lid 9. The lid 9 of the cell 1a is provided with the nozzle 18 for recovering desorbed carbon dioxide. The lid 9 of the cell 1b is provided with the gas-flushing inlet 11 for introducing an inert gas such as nitrogen (N₂) and the gas-flushing outlet 12 for exhausting oxygen (O₂) and other gases that may have been remaining in the electrolytic solutions 5a and 5b, which can be bubbled out by a flushing procedure prior to introducing the gas containing CO₂. Specifically, N₂ can be introduced into the electrolytic solution 5b in the cell 1b through the gas-flushing inlet 11 with the bottom end thereof submerged into the electrolytic solution 5b, as illustrated, whereby bubbling can be performed. O₂ and other gases remaining in the electrolytic solutions 5a and 5b can be expelled by the bubbling, and exhausted out from the cells 1a and 1b respectively through the nozzle 18 and gas-flushing outlet 12. After eliminating the remaining gas by bubbling, the gas-flushing inlet 11 can be drawn out from the electrolytic solution 5b (not illustrated). N₂ can be continually introduced into the cell 1b through the gas-flushing inlet 11 and exhausted out through the gas-flushing outlet 12 to provide a N₂ flow, so as to prevent external oxygen from entering the system during the CO₂ absorption-desorption operation. The potential of the working electrode 2 can be adjusted to a potential where CO₂ adsorbs to the carbon dioxide adsorbent in the electrolytic solution 5a, while the gas containing CO₂ is introduced through the nozzle 8, whereby the contained CO₂ can adsorb to the carbon dioxide adsorbent. The remaining gas in the cell 1a after CO₂ has been recovered therefrom can then be exhausted through the nozzle 18. The potential of the working electrode 2 can then be switched to a potential where CO₂ desorbs from the carbon dioxide adsorbent, and the CO₂ can be recovered from the nozzle 18. Valves for opening and closing the flow paths of the nozzle 8, gas-flushing inlet 11, gas-flushing outlet 12 and nozzle 18 are provided, although not shown in drawing. In the illustrated example, the working electrode 2 and the counter electrode 3 are separately located in the cell 1a and the cell 1b, respectively. The arrangement of the electrodes is not limited to this example, and for example, the counter electrode and the working electrode may be integrated with the separator interposed therebetween. A specific example is an electrochemical cell 120 illustrated in FIG. 8.

The electrochemical cell 120 illustrated in FIG. 8 includes a cell 121, a working electrode 122, a counter electrode 123, and a separator 126. The cell 121 is divided into upper and lower two chambers with the separator 126 interposed therebetween, and the working electrode 122 and the counter electrode 123 are provided on the upper and lower principal surfaces of the separator 126, respectively. An electrolytic solution 125 is contained in the lower chamber of the cell 121, and the counter electrode 123 is in contact with the electrolytic solution 125. The counter electrode 123 may be immersed in the electrolytic solution 125. The working electrode 122 is located in the upper chamber of the cell 121. The working electrode 122 includes a carbon dioxide adsorbent, and may include the porous composite described above, for example. The electrolytic solution 125 contained in the lower chamber does not include an adsorbent. The upper chamber in which the working electrode 122 is present can function as a gas flow path 128, and a gas containing carbon dioxide is passed through the gas flow path 128 so that the contained carbon dioxide can be adsorbed onto the adsorbent in the working electrode 122.

A porous collector electrode 124 may be stacked on the outer side of the working electrode 122 in order to improve power distribution to the working electrode 122, and a porous support 127 for fixing the whole of the working electrode 122 and the porous collector electrode 124 may be stacked on the further outer side thereof. Similarly, the support 127 made of a porous material may be stacked on the outside of the counter electrode 123, as well. The end portion of the thus fabricated stack of the porous collector electrode 124, the working electrode 122, the separator 126, and the counter electrode 123 sandwiched between the supports 127 can be fixed by fixtures 129. Since the working electrode 122 is porous, the porous collector electrode 124 may be omitted as necessary. When the mechanical strengths of the working electrode 122 and the counter electrode 123 are sufficient, the porous support 127 can be omitted. Although not illustrated, the electrochemical cell 120 may also be provided with an inlet and outlet used for bubbling out remaining gas from the electrolytic solution 125 and providing a N₂ flow, as well.

Hereinafter, the current collector, the electro-conductive member, and the electrolytic solution will be described in detail.

### Current Collector

As the current collector, a member made of carbon or metal may be used. With the current collector having better electrical conductivity and a larger surface area, charges can be transferred to more Red-Ox active moieties. Examples of carbon-made members include glassy carbon, a graphite sheet, carbon felt, carbon cloth, a carbon mesh, carbon paper, and a carbon sheet with a gas diffusion layer. Examples of the metal member include a copper plate, a copper sheet, a copper mesh, an aluminum plate, an aluminum sheet, an aluminum mesh, a nickel plate, a nickel sheet, and a nickel mesh. The carbon members and metal members are not limited to the above.

### Electro-conductive Member

As the electro-conductive member, for example, a carbon material having good electrical conductivity can be used. Examples of the electro-conductive member include one or more selected from the group consisting of carbon nanotube, graphite, graphene, carbon nanofiber, and Ketjen black. The shape of the electro-conductive member is preferably linear or flat in order to increase the contact probability, and is preferably a rod shape, a tubular shape, a fiber shape, a sheet shape, or a flake shape.

### Electrolyte

The electrolyte may be a liquid electrolyte (electrolytic solution), for example. The electrolyte includes an ionically bonded salt, for example. Specifically, the ionically bonded salt is preferably one or more selected from the group consisting of an alkali metal salt, an alkaline earth metal salt, a transition metal salt, an amphoteric metal salt, an ammonium salt, an imidazolium salt, a pyridinium salt, and a phosphonium salt. The form of the ionic-bonding salt may be a solid or a liquid, and examples of the liquid include an ionic liquid.

As the cation of the ionically bonded salt, one or more selected from the group consisting of alkali metals such as Li, Na, K, Rb, and Cs; alkaline earth metals such as Mg, Ca, Sr, and Ba; transition metals such as Ti, Cr, Mn, Fe, Co, Ni, Cu, Zn, Zr, Pd, and Ag; amphoteric metals such as Al, Ga, and Sn; ammonium ion and ammonium ion substituted with C1 to C5 lower hydrocarbons (N(CₙH₂ₙ₊₁)₄⁺, n = 1 to 5); imidazolium ions or imidazolium ions with 1 to 3 substituents; pyridinium ions or pyridinium ions with 1 to 3 substituents; piperidinium ions or piperidinium ions with 1 to 3 substituents; pyrrolidinium ions or pyrrolidinium ions with 1 to 3 substituents; phosphonium ions PR₄⁺ (R is hydrocarbon group) with four substituents may be used, and one of the above may be used alone, or plural of the above may be used in combination.

As the anion of the ionic-bonding salt, one or more selected from the group consisting of halogens such as Cl-, Br⁻, and I⁻, nitrate ion (NO₃⁻), PF₆⁻, BF₄⁻, CF₃SO₃⁻, N(SO₂F₂)₂⁻, N(SO₂CF₃)₂⁻, CH₃COO⁻, CF₃COO⁻, and ClO₄⁻ may be used, and one of the above may be used alone, or plural of the above may be used in combination.

As the ion conductive polymer, for example, one or more selected from the group consisting of polyethylene oxide (PEO), polypropyl oxide (PPO), polyacrylonitrile (PAN), polyvinyl chloride (PVC), an ionic liquid polymer, polyaniline, and polythiophene (PEDOT) are preferably used.

The electrolytic solution may contain a solvent. The solvent of the electrolytic solution may be water or an organic solvent, for example. If the electrolyte is an ionic liquid, a solvent may be used or omitted. As the organic solvent, it is desirable to use one or more selected from the group consisting of acetonitrile, propylene carbonate, ethylene carbonate, vinylene carbonate, vinylethylene carbonate, dimethyl carbonate, diethyl carbonate, ethyl methyl carbonate, succinic anhydride, cyclohexylbenzene, thiophene, toluene, fluorobenzene, and hexafluorobenzene.

### <Manufacturing Method>

The carbon dioxide adsorption-desorption device can be manufactured as follows, for example.

First, a carbon dioxide adsorbent is prepared. For example, an adsorbent is obtained by synthesis.

In order to obtain a device including the adsorbent in a solution state, the prepared adsorbent can be added to the electrolytic solution, and the electrode can be set so as to be in contact with the obtained solution.

When the adsorbent is integrated with the electrode, for example, a porous material containing the adsorbent is synthesized, and a paste containing the obtained porous material is applied onto the current collector to obtain an electrode containing the adsorbent. Specifically, the synthesized porous material is mixed as appropriate with an electro-conductive member, a binder, a solvent, and the like to prepare the paste. This paste is applied onto the current collector to obtain an electrode.

The above is merely an example, and the paste need not necessarily contain a binder. Depending on the properties of the electro-conductive member, the paste may be made into a free-standing electrode by heating and drying. Namely, an electrode including a porous material containing an adsorbent alone may be obtained, for example, without using the current collector.

When an electrolyte is to be included in the electrode, for example, an electrolyte solution is prepared, the prepared solution is cast onto the electrode surface, and then vacuum impregnation is performed, whereby the electrolyte can be included within the electrode.

The carbon dioxide adsorption-desorption device described above includes a carbon dioxide adsorbent having a fused heteroaromatic ring structure. The fused heteroaromatic ring structure includes one or more quinone structures. Such a device is a carbon dioxide adsorption-desorption device capable of efficiently adsorbing and desorbing carbon dioxide with low energy.

### (Carbon Dioxide Separation Method)

A carbon dioxide separation method according to an approach includes, by using the carbon dioxide adsorption-desorption device described above, removing carbon dioxide from a gas containing the carbon dioxide by adsorbing the carbon dioxide onto an adsorbent, and regenerating the adsorbent by desorbing the carbon dioxide from the adsorbent. Adsorbing the carbon dioxide onto the adsorbent includes causing an electrical current to flow through the adsorbent at the reduction potential of the adsorbent, and bringing the gas containing the carbon dioxide into contact with the adsorbent in a state where a potential flowing through the adsorbent is maintained at the reduction potential. Regenerating the adsorbent by desorbing the carbon dioxide from the adsorbent includes switching the potential flowing through the adsorbent that had adsorbed carbon dioxide to the oxidation potential of the adsorbent. The regenerated adsorbent can be reused.

By setting the device again to the reduction potential after the separation of the carbon dioxide from the adsorbent,the device is again able to perform the adsorption treatment of carbon dioxide. In this way, the device can be repeatedly used numerous times by performing switching of the potential of electrical current flowing through the adsorbent between the reduction potential and the oxidation potential.

The method for bringing the gas containing carbon dioxide into contact with the adsorbent is not particularly limited. For example, for a carbon dioxide adsorption-desorption device including an electrolytic solution, there is a method by which a gas containing carbon dioxide is bubbled in the electrolytic solution to cause the carbon dioxide to be adsorbed in the electrolytic solution. As another example, there is a method by which the carbon dioxide adsorption-desorption device is configured to expose the adsorbent to a gas flow containing carbon dioxide.

The concentration of the carbon dioxide to be brought into contact with the adsorbent is not particularly limited, but may correspond to a wide range of CO₂ concentrations from an atmospheric level to a level of exhausts from a thermal power plant or the like. Specifically, the concentration of the carbon dioxide is preferably 0.01 vol% or more, and more preferably 0.04 vol% or more to 50 vol% or less.

The environmental temperature at the time of the carbon dioxide adsorption and desorption treatment is usually preferably 0°C or more to 60°C or less. The temperature is more preferably 50°C or lower, and particularly preferably 10°C or more to 45°C or less. The adsorption amount of carbon dioxide increases at a lower temperature. The lower limit value of the treatment temperature can be determined according to the gas temperature in the process, the heat recovery target, and the like. The adsorption pressure of carbon dioxide is usually approximately equal to the atmospheric pressure. Higher pressure may also be applied in order to increase adsorption performance.

The method for adsorbing and desorbing carbon dioxide using the carbon dioxide adsorption-desorption device may include, for example, the following steps of:
applying current at reduction potential to a moiety containing the adsorbent;
adsorbing carbon dioxide onto the adsorbent;
flowing electrical current with the potential changed to the oxidation potential; and
desorbing carbon dioxide from the adsorbent.

The carbon dioxide separation method described above includes causing carbon dioxide to be adsorbed onto the adsorbent included in the carbon dioxide adsorption-desorption device, causing the adsorbent that had adsorbed the carbon dioxide to desorb and separate the carbon dioxide, and regenerating the adsorbent. The carbon dioxide is caused to be adsorbed onto the adsorbent in a state where the electrical current flowing through the adsorbent is maintained at the reduction potential. In a state where the electrical current flowing through the adsorbent is switched to the oxidation potential then the oxidation potential is maintained, the carbon dioxide is desorbed. According to this separation method, carbon dioxide can be efficiently adsorbed and desorbed with low energy.

### Examples

Hereinafter, examples of a carbon dioxide adsorption-desorption device using the carbon dioxide adsorbent will be described.

### (Example 1)

### <Preparation of Phenanthrene-dithiophene (PDT)>

Commercially available phenanthrene-dithiophene (PDT) was prepared and used as it was.

### <Production of Device>

The PDT and tetrabutylammonium tetrafluoroborate (TBABF₄) as an electrolyte were added to propylene carbonate (PC) at respective concentrations of 0.2 mol/L and 0.75 mol/L, and heated and mixed (about 80°C) to obtain a pale yellow transparent solution. The obtained solution was set as an electrolytic solution in an electrolytic cell to obtain a device of Example 1. Other details of the device will be described below.

### (Example 2)

### <Preparation of 2,6-diaminobenzo[1,2-b:4,5-b']dithiophene-4,8-dione (2NH₂-BDTD)>

Commercially available 2,6-diaminobenzo[1,2-b:4,5-b']dithiophene-4,8-dione (2NH₂-BDTD) was prepared and used as it was.

### <Production of Device>

The 2NH₂-BDTD and TBABF₄ were added to PC, and adjusted so as to be 0.2 mol/L and 0.75 mol/L, respectively, to obtain an electrolytic solution. The obtained electrolytic solution was set in an electrolytic cell to obtain a device of Example 2. Other details of the device will be described below.

### (Example 3)

### <Synthesis of 2NH₂-BDTD-TFP-COF Composite Electrode>

Referring to the non-patent document J. Am. Chem. Soc., 2013, 135, 16821-16824, 2NH₂-BDTD and 1,3,5-triformylphloroglucinol (TFP) were placed in a heat-resistant container at a molar ratio of 1:3, and heated with a dioxane solvent at 120°C for two days. The obtained crude crystals were washed with a dimethylacetamide (DMA) solvent, filtered, and dried under reduced pressure to obtain 2NH₂-BDTD-TFP-COF. The obtained 2NH₂-BDTD-TFP-COF was mixed with carbon nanofibers (CNF) and polyvinylidene fluoride (PVdF) at a mass ratio of 6:3:1 under an N-methylformaldehyde solvent (NMF solvent), and applied onto a carbon felt to obtain an electrode as a composite containing a porous material.

### <Production of Device>

The composite electrode obtained above was set in an electrolytic cell, and a device of Example 3 was obtained using an electrolytic solution prepared by dissolving TBABF₄ in PC at 0.75 mol/L. Other details of the device will be described below.

### (Example 4)

### <Preparation of 5,8-Quinolinediol (QD)>

Commercially available 5,8-quinolinediol (QD) was prepared and used as it was.

### <Production of Device>

The QD and TBABF₄ were added to PC, and adjusted so as to be 0.2 mol/L and 0.75 mol/L, respectively, to obtain an electrolytic solution. The obtained electrolytic solution was set in an electrolytic cell to obtain a device of Example 4.

### (Example 5)

### <Preparation of Benzo[1,2-b:4,5-b']difuran-4,8-dione (BDFD)>

Commercially available benzo[1,2-b:4,5-b']difuran-4,8-dione (BDFD) was prepared and used as it was.

### <Production of Device>

The BDFD and TBABF₄ were added to PC, and adjusted so as to be 0.2 mol/L and 0.75 mol/L, respectively, to obtain an electrolytic solution. The obtained electrolytic solution was set in an electrolytic cell to obtain a device of Example 5.

### (Example 6)

### <Preparation of 1H-Indole-4,5-dione (ID)>

Commercially available 1H-indole-4,5-dione (ID) was prepared and used as it was.

### <Production of Device>

The ID and TBABF₄ were added to PC, and adjusted so as to be 0.2 mol/L and 0.75 mol/L, respectively, to obtain an electrolytic solution. The obtained electrolytic solution was set in an electrolytic cell to obtain a device of Example 6.

### (Example 7)

### <Preparation of Benzo[b]selenophene-4,7-dione (BSD)>

Commercially available benzo[b]selenophene-4,7-dione (BSD) was prepared and used as it was.

### <Production of Device>

The BSD and TBABF₄ were added to PC, and adjusted so as to be 0.2 mol/L and 0.75 mol/L, respectively, to obtain an electrolytic solution. The obtained electrolytic solution was set in an electrolytic cell to obtain a device of Example 7.

### (Comparative Example 1)

### <Preparation of 1,8-Diaminoanthraquinone (2NH₂-AQ)>

Commercially available 1,8-diaminoanthraquinone (2NH₂-AQ) was prepared and used as it was.

### <Production of Device>

A device of Comparative Example 1 was obtained in the same manner as in Example 1 except that the 2NH₂-AQ was used instead of PDT.

### (Comparative Example 2)

### <Preparation of Naphthazarin (NZ)>

Naphthazarin (NZ) was obtained.

### <Production of Device>

A device of Comparative Example 2 was obtained in the same manner as in Example 1 except that the NZ was used instead of PDT.

### (Evaluation)

### <Infrared Spectroscopy>

The PDT prepared in Example 1 was subjected to infrared spectroscopy. Infrared spectroscopy was performed as follows. An infrared spectrometer (FT-IR6100 manufactured by JASCO Corporation) was used, and measurement was performed in a measurement range of 400 cm⁻¹ to 4000 cm⁻¹ at a measurement rate of 40 cm⁻¹/sec. Absorption derived from stretching vibration of an aromatic S-C bond was observed in the vicinity of 1100 cm⁻¹, absorption derived from stretching vibration of an aromatic ring was observed in the vicinity of 1400 cm⁻¹ to 1600 cm⁻¹, and strong absorption derived from stretching vibration of a C=O bond was observed in the vicinity of 1600 cm⁻¹ to 1850 cm⁻¹, and therefrom, a heteroaromatic ring structure containing S and having a quinone structure was confirmed.

### <Raman Spectroscopy>

The PDT prepared in Example 1 was subjected to Raman spectroscopy.

The Raman spectroscopy was performed as follows. The PDT was subjected to a measurement using a Raman spectrometer (LabRAM Evolution manufactured by HORIBA) in a measurement range of 200 cm⁻¹ to 3300 cm⁻¹ at an excitation laser wavelength of 532 nm. Strong emission derived from the stretching vibration of the aromatic S-C bond was observed at 1060 cm⁻¹ to 1100 cm⁻¹, and strong emission derived from the aromatic ring stretching vibration was observed at 1400 cm⁻¹ to 1600 cm⁻¹, and therefrom, a heteroaromatic ring structure containing S within the molecular structure was confirmed.

### <NMR Measurements>

The PDT prepared in Example 1 was subjected to ¹H and ¹³C NMR measurements. The measurement conditions and the peaks observed in the measurement spectrum were as follows.

¹H NMR (CD₂Cl₂, 400 MHz, δ in ppm) : 7.62 (dd, 2H), 7.44(dd, 2H) .

¹³C NMR (DMSO, 100 MHz, δ in ppm) :127.0, 135.0, 138.2, 149.5, 177.0.

### <Electrochemical Measurements>

Each of the devices produced in Examples 1 to 6 and Comparative Examples 1 and 2 was evaluated for electrochemical performance. Specifically, a test device using each of the produced devices was constructed and subjected to cyclic voltammetry (CV) measurement.

In Examples 1, 2, and 4 to 6 and Comparative Examples 1 and 2, a three-electrode electrochemical cell configuration similar to that of the carbon dioxide adsorption-desorption device 100 illustrated in FIG. 7 was adopted for the test device. In the test device, a 3 mm glassy carbon rod was used as the working electrode 2, an electrolytic solution containing the adsorbent prepared in each of Examples 1, 2 and 4 to 6 and Comparative Examples 1 and 2 was used as a working electrode-side electrolytic solution (5a), a platinum mesh electrode was used as the counter electrode 3, a 0.01 mol/L TBABF₄ electrolytic solution containing 0.75 mol/L ferrocene was used as a counter electrode-side electrolytic solution (5b), and an Ag/Ag⁺ electrode (acetonitrile/0.1 M (M: mol/L) tetrabutylammonium perchlorate solution/0.01 M AgNO₃) was used as a reference electrode 4.

Before the measurement, argon (Ar) gas was passed through a nozzle 8 into the electrolytic solution 5a within a cell 1a for about 30 minutes, and CV measurement was performed using a potentiostat 10. Subsequently, 100% carbon dioxide gas was passed through the nozzle 8 into an electrolytic solution 5a for about 30 minutes, and CV measurement was performed again.

For Example 3, since the adsorbent was integrated with the electrode, measurement was performed with a test device using an electrochemical cell having the configuration shown in FIG. 8. The prepared composite electrode was used as a working electrode 122, and was stacked on a platinum mesh electrode as a counter electrode 123 with a separator 126 interposed therebetween. In order to improve power distribution to the composite electrode, a porous collector electrode 124 was stacked on the outside of the working electrode 122, and a porous support 127 for fixing the entire electrode was stacked further on the outer side thereof. Similarly, a support 127 made of a porous material was stacked on the outside of the counter electrode 123, and the ends were fixed by fixtures 129. An electrolytic solution 125 (0.01 mol/L TBABF₄ solution containing 0.75 mol/L ferrocene without adsorbent) was put into one chamber of a cell 121 having two communicating chambers, and the stack including the working electrode 122 and the counter electrode 123 was set in the cell 121 so as to block the communication between the two chambers and to immerse the counter electrode 123 in the electrolytic solution 125. The chamber into which the electrolytic solution 125 was not put was used as a gas flow path 128 through which Ar gas and carbon dioxide gas were allowed to pass. The separator 126 used had a structure in which the electrolytic solution 125 permeates into the working electrode 122 through the separator 126. CV measurement was performed in the same manner as in Example 1 and the like except that such an electrochemical cell 120 was used.

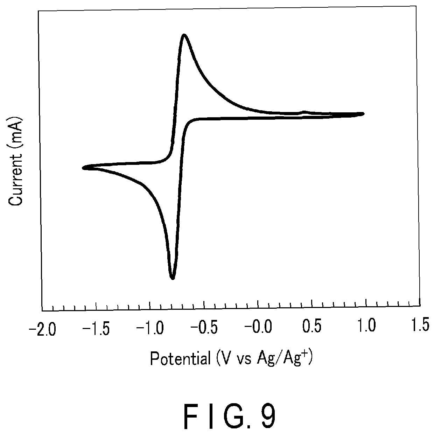

As an example, FIG. 9 shows the CV measurement results of PDT obtained by the device of Example 1. In the graph of FIG. 9, a curve during the CV measurement under a CO₂ atmosphere is indicated by a solid line. As shown in the graph, a reduction peak around -0.8 V (vs. Ag/Ag⁺) and an oxidation peak around -0.66 V (vs. Ag/Ag⁺) were observed, and thus, an oxidation-reduction reaction was confirmed at a potential higher than the oxidation-reduction potential of oxygen, which coincided with the expectation by calculation.

### <CO₂ Adsorption/Desorption Test>

A carbon dioxide adsorption/desorption test was performed using the devices produced in each of Example 1 to 6 and Comparative Examples 1 and 2. The basic operations of the test were generally the same as the CV evaluation. First, an inert gas was introduced into an electrolytic solution, and bubbling was carried out for about 30 minutes. As the inert gas, argon gas was used. Thereafter, while bubbling carbon dioxide gas, a potential slightly exceeding the reduction potential of each adsorbent was applied for a set period of time to adsorb carbon dioxide to the adsorbent. Thereafter, the space above the electrolytic solution was flushed with an inert gas, and a tube for recovering CO₂ was connected to the cell. The carbon dioxide was desorbed from the adsorbent by applying a potential slightly exceeding the oxidation potential of each adsorbent for a set period of time in a state where all portions except the CO₂ recovery tube were sealed. The desorbed carbon dioxide accumulated in the space above the electrolytic solution. The application of the potential for desorbing the carbon dioxide was stopped, and then sampling was performed on the space above the electrolytic solution. The collected gas was used to perform quantification by gas chromatography (GC), and the amount of carbon dioxide adsorbed and recovered by the adsorbent was determined. Table 5 below shows the amount of CO₂ recovered by each adsorbent. Table 5 also shows the abbreviation and chemical structure of each adsorbent. However, the chemical structure of Example 3 is shown below Table 5 due to its large size.

**[Table 5]**

| Amounts of CO₂ Recovered in Examples and Comparative Examples | | | |
|---|---|---|---|
| | Adsorbent | Chemical structure of adsorbent | Amount of CO₂ recovered (mmol/g) |
| Example 1 | PDT | 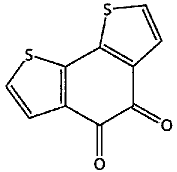 | 2.5 |
| Example 2 | 2NH₂-BDTD | 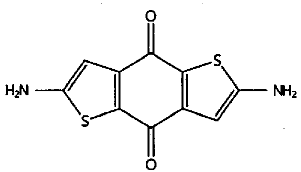 | 2.3 |
| Example 3 | 2NH₂-BDTD-TFP-COF | (Shown outside Table 5) | 2.6 |
| Example 4 | QD | 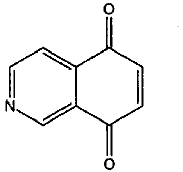 | 2.9 |
| Example 5 | BDFD | 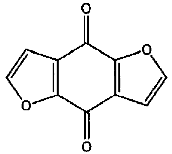 | 2.3 |
| Example 6 | ID | 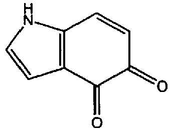 | 2.4 |
| Example 7 | BSD | 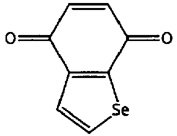 | 2.2 |
| Comparative Example 1 | 2NH₂-AQ | 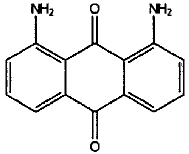 | 1.1 |
| Comparative Example 2 | NZ | 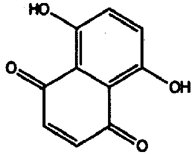 | 1.5 |

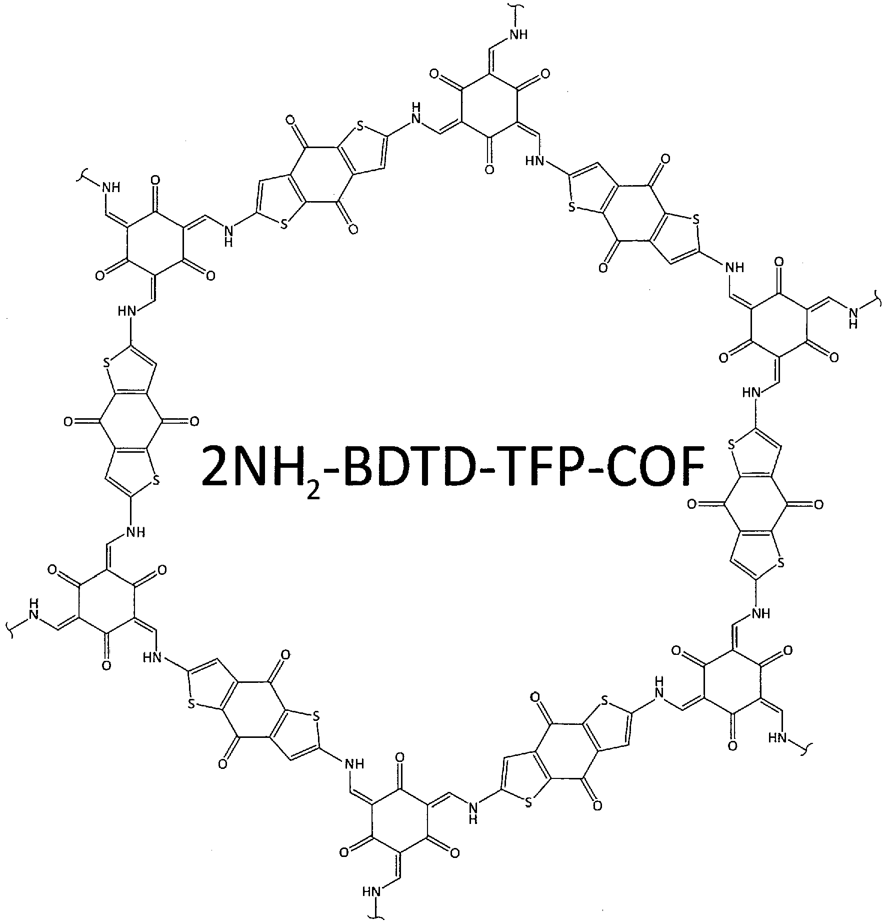

From the results of Examples 1 to 6 and Comparative Examples 1 and 2 shown in Table 5, it can be seen that there is an advantage in forming a heterocyclic ring by partially replacing carbon constituting the ring and not just simply introducing a substituent to the outer periphery of the condensed ring. From Examples 1 to 3, it can be seen that a significant difference in the performance of the adsorbent having a fused heteroaromatic ring structure does not arise depending on the heteroatoms introduced into the ring. It can further be seen from Examples 2 and 3 that with COF, since the COF was disposed onto the electrode, the exchange of electrons was easier than that for the solution type, and the amount of CO₂ had slightly increased.

According to one or more approach or example described above, a carbon dioxide adsorbent and a carbon dioxide adsorption-desorption device using the same are provided. The carbon dioxide adsorbent has a fused heteroaromatic ring structure that includes one or more quinone structures, and is able to efficiently adsorb and desorb carbon dioxide. Accordingly, the device is able to efficiently adsorb and desorb carbon dioxide, and can perform efficient recovery of carbon dioxide.

While certain arrangements have been described, these arrangements have been presented by way of example only, and are not intended to limit the scope of the inventions. Indeed, the carbon dioxide adsorbents and carbon dioxide adsorption-desorption devices described herein may be embodied in a variety of other forms; furthermore various omissions, substitutions and changes in the form of the materials and apparatuses described herein may be made.

The present disclosure also encompasses the following approaches relevant to a carbon dioxide adsorbent and carbon dioxide adsorption-desorption device:
1. A carbon dioxide adsorbent which is capable of adsorbing and desorbing carbon dioxide, and has a fused heteroaromatic ring structure that comprises one or more quinone structures.
2. The carbon dioxide adsorbent according to clause 1, wherein the carbon dioxide adsorbent can adsorb carbon dioxide to the quinone structure in reduction state, and the carbon dioxide adsorbent can desorb carbon dioxide from the quinone structure in oxidation state.
3. The carbon dioxide adsorbent according to clause 1 or 2, wherein the carbon dioxide adsorbent is able to perform oxidation-reduction reaction by electrical response.
4. The carbon dioxide adsorbent according to any one of clauses 1 to 3, wherein the fused heteroaromatic ring structure has one or more selected from the group consisting of a benzene-based heteroaromatic ring structure, a naphthalene-based heteroaromatic ring structure, an anthracene-based heteroaromatic ring structure or a phenanthrene-based heteroaromatic ring structure.
5. The carbon dioxide adsorbent according to any one of clauses 1 to 4, wherein the fused heteroaromatic ring structure includes one or more selected from the group consisting of nitrogen, oxygen, and sulfur.
6. The carbon dioxide adsorbent according to any one of clauses 1 to 5, wherein the carbon dioxide adsorbent is a porous metal-organic framework.
7. The carbon dioxide adsorbent according to any one of clauses 1 to 5, wherein the carbon dioxide adsorbent is a porous covalent-organic framework.
8. The carbon dioxide adsorbent according to any one of clause 1 to 5, wherein the carbon dioxide adsorbent is a polymer including a molecule containing the cyclic disulfide structure as a unit structure.
9. The carbon dioxide adsorbent according to any one of clauses 1 to 8, wherein the carbon dioxide adsorbent exhibits a higher oxidation-reduction potential than that of oxygen.
10. A carbon dioxide adsorption-desorption device comprising the carbon dioxide adsorbent according to any one of clauses 1 to 9.

## Claims

1. A carbon dioxide adsorbent which is capable of adsorbing and desorbing carbon dioxide, and comprises a fused heteroaromatic ring structure that includes one or more quinone structures.

2. The carbon dioxide adsorbent according to claim 1, wherein the carbon dioxide adsorbent can adsorb carbon dioxide to the quinone structure in reduction state, and the carbon dioxide adsorbent can desorb carbon dioxide from the quinone structure in oxidation state.

3. The carbon dioxide adsorbent according to claim 1 or 2, wherein the carbon dioxide adsorbent is able to perform oxidation-reduction reaction by electrical response.

4. The carbon dioxide adsorbent according to any one of claims 1 to 3, wherein the fused heteroaromatic ring structure has one or more selected from the group consisting of a benzene-based heteroaromatic ring structure, a naphthalene-based heteroaromatic ring structure, an anthracene-based heteroaromatic ring structure, and a phenanthrene-based heteroaromatic ring structure.

5. The carbon dioxide adsorbent according to any one of claims 1 to 4, wherein the fused heteroaromatic ring structure contains one or more selected from the group consisting of nitrogen, oxygen, and sulfur.

6. The carbon dioxide adsorbent according to any one of claims 1 to 5, wherein the carbon dioxide adsorbent is a porous metal-organic framework.

7. The carbon dioxide adsorbent according any one of claims 1 to 5, wherein the carbon dioxide adsorbent is a porous covalent-organic framework.

8. The carbon dioxide adsorbent according to any one of claims 1 to 5, wherein the carbon dioxide adsorbent is an oligomer or a polymer including molecules containing the fused heteroaromatic ring structure as a unit structure.

9. The carbon dioxide adsorbent according to any one of claims 1 to 8, wherein the carbon dioxide adsorbent exhibits a higher oxidation-reduction potential than that of oxygen.

10. A carbon dioxide adsorption-desorption device (100) comprising the carbon dioxide adsorbent according to any one of claims 1 to 9.
